Europäisches Patentamt

European Patent Office

Office européen des brevets

(11) Publication number: **0 247 543**
**A1**

## (12) EUROPEAN PATENT APPLICATION

(21) Application number: **87107509.9**

(22) Date of filing: **22.05.87**

(51) Int. Cl.⁴: **A61B 5/02** , G01L 7/08

(30) Priority: **26.05.86 JP 120915/86**

(43) Date of publication of application:
**02.12.87 Bulletin 87/49**

(84) Designated Contracting States:
**BE DE FR GB SE**

(71) Applicant: **Terumo Kabushiki Kaisha**
**No. 44-1, Hatagaya 2-chome Shibuya-ku**
**Tokyo 151(JP)**

(72) Inventor: **Moriuchi, Yousuke**
**4020-18, Mannoharashinden Fujinomiya-shi**
**Shizuoka-ken(JP)**

(74) Representative: **Henkel, Feiler, Hänzel &**
**Partner**
**Möhlstrasse 37**
**D-8000 München 80(DE)**

(54) Liquid pressure transducer dome.

(57) A liquid pressure transducer dome (10) includes a dome body (12) provided with means (22a) for ensuring its connection to a liquid pressure transducer, a diaphragm (16) which comprises a film which is pervious to gas but impervious to liquids, and is set in the dome (10) to transmit liquid pressure to the liquid pressure transducer, and liquid passage means (14, 20), provided in the dome, for conducting the liquid to the diaphragm (16). The diaphragm (16) discharges gases present in the liquid passage means (14, 20) therethrough, when a liquid is introduced into the dome body.

F I G. 1

Xerox Copy Centre

# Liquid pressure transducer dome

This invention relates to a liquid pressure transducer dome which transmits the pressure of a liquid, particularly blood, to a liquid pressure transducer.

A pressure transducer is applied to the patient, in order to measure blood pressure. In this case, a catheter is held in the patient's blood vessel. The proximal end of the catheter is connected to a pressure transducer through a pressure transmission tube and pressure transducer dome (pressure dome). The pressure transmission tube and pressure dome are filled with a liquid such as physiological saline solution. The blood pressure is transmitted through the liquid held in the tube, and the pressure dome to the pressure transducer through a diaphragm disposed over the bottom plane of the dome. The pressure transducer converts the received blood pressure into an electric signal.

In the above-mentioned blood pressure measuring process, it is necessary to previously subject the catheter, pressure transmission tube, and pressure dome to priming by means of a liquid, to expel air therefrom. If any air remains in these members, this air acts as a sort of cushion and varies the frequency characteristic of the blood pressure, thus preventing accurate measurement of blood pressure.

The conventional priming process is to introduce a liquid at an inlet provided at the top of the pressure dome and expel air by the liquid through an air outlet formed at the other position of the dome. In the conventional semispherical pressure dome, air bubbles settle on the walls of the pressure dome and diaphragm surface.

For the resolution of the above-mentioned problems, USP 4,462,409 discloses a dome body which is not shaped like a semispherical form but partially assumes a convex-tapered form for the object of facilitating the release of retained air. Yet this dome still fails to give full satisfaction.

Further, Japanese Patent Disclosure 56-97435 (Kokai) proposes a structure which is designed in a way to give rise to the vertical flow of a liquid in the dome, thereby easily pushing upward air bubbles attached to dome walls to the top portion thereof. However, this proposed device has a undesirable structural complexities.

It is an object of this invention to provide a liquid pressure transducer dome which has a simple structure and facilitates air release. A liquid pressure transducer dome according to the present invention has a dome body provided with means for effecting its connection to a liquid pressure transducer, a diaphragm provided in the dome body, and fluid-passage means which allows the introduction of the fluid up to the diaphragm. The diaphragm is prepared from a porous film which is pervious to gas but impervious to liquids. When a liquid enters the transducer dome, air present in the fluid passage means is discharged off through the diaphragm.

The central portion of the diaphragm should preferably be rendered transparent or translucent. Generally the diaphragm is prepared from hydrophobic materials such as polyethylene, polypropylene, polytetrafluoroethylene, polyvinylidene fluoride or tetrafluoroethylene-hexafluoropropylene copolymer. The diaphragm is preferred to have an average pore diameter ranging between 0.01 to 5 μm.

The liquid pressure transducer dome embodying this invention need not be constructed in the undermentioned dome-like (namely, the so-called semispherical) form; it may be constituted in any other form, provided it attains the intended object.

This invention can be more fully understood from the following detailed description when taken in conjunction with the accompanying drawings, in which:

Fig. 1 is a sectional view of a liquid pressure transducer dome according to a first embodiment of this invention;

Fig. 2 is a plan view of another diaphragm used in the liquid pressure transducer dome of the invention; and

Fig. 3 is a sectional side view of a dome using a liquid pressure transducer according to another embodiment of the invention.

The present inventor has noticed that the conventional liquid pressure transducer dome makes use of a diaphragm impervious to both fluids and gas, and consequently the pressure dome has to be provided with a liquid inlet and gas outlet, and found that the application of a porous diaphragm pervious to gas (air), but impervious to a liquid can provide a liquid pressure transducer dome which facilitates the withdrawal of air and is of simple structure.

A liquid pressure transducer dome embodying the present invention will now be described, with reference to the appended drawings. Throughout the drawings, the same parts are denoted by the same numerals.

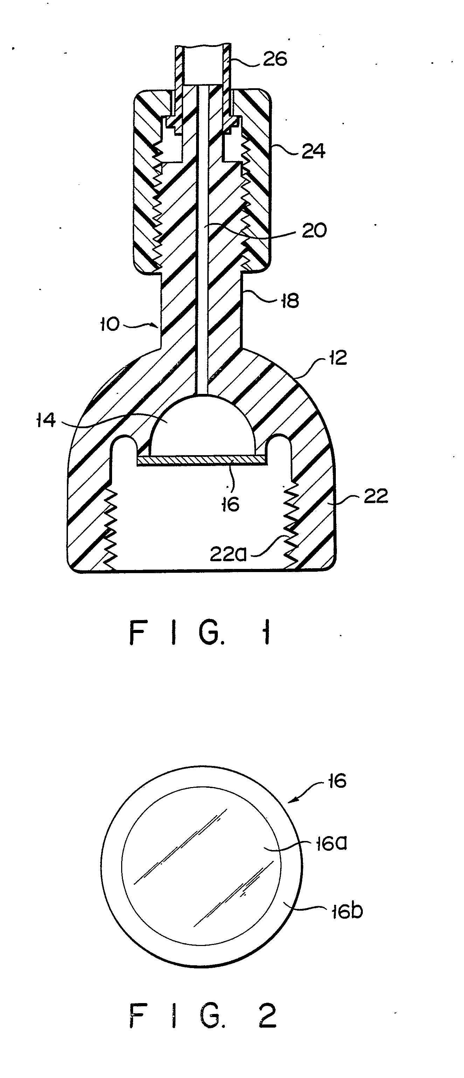

In Fig. 1 pressure transducer dome 10 of the present invention has a semispherical body 12. The bottom of dome 10 is fitted with circular diaphragm 16 which closes opening 14 at the bottom wall of dome 10.

Diaphragm 16 is prepared from hydrophobic, porous material which is pervious to gas, but impervious to liquids. The diaphragm membrane can be prepared from hydrophobic material such as polyethylene, polypropylene, polytetrafluoroethylene, polyvinylidene fluoride, and tetrafluoroethylene-hexafluoropropylene copolymer. Of the above materials polytetrafluoroethylene is the most preferred.

The diameter of the pores of diaphragm 16 should preferably fall within the range of 0.01 and 5 $\mu$m on average, more preferably between 0.05 and 2 $\mu$m, and most preferably between 0.08 and 0.5 $\mu$m. From the point of view of mechanical strength, the pore diameter should preferably be about 0.1 $\mu$m on average.

In this case it should be noted that the pore diameter is determined by the step of optionally selecting about 500 pores by means of, for example, an electronic microscope, and defining the average value as the average pore diameter. If the pores are elliptic, the sum of the lengthwise and crosswise diameters are divided by 2 and the elliptic pores are regarded as circles having a diameter represented by the quotient. The average pore diameter may also be determined by mercury porosimetry.

The desired porosity of diaphragm 16 is to be 30% or more, preferably 50% or more, and most preferably 60% or more. The porosity is determined by the following steps. The diaphragm is cut up in a predetermined size (represented by A). The weight of a void-free cut piece of the membrane bearing the above-mentioned size is calculated from the specific gravity of the membrane and its volume (B) by the undermentioned formula:

$$\frac{B - A}{B} \times 100 \quad (porosity)$$

Diaphragm 16 may be fitted to dome body 12 by means of forced insertion, ultrasonic fusion or caulking which involves the application of an O ring or flat rubber packing. The simplest means for the attachment of diaphragm 16 is by thermal fusion.

The center of the head portion of dome 12 is provided with liquid inlet pipe 18 which constitutes liquid passage 20 allowing for the introduction of a liquid into interior 14 of dome 12. Liquid-conducting tube 26 coupled to a catheter is connected to pipe 18. Tube 26 is fixed in position by means of threaded engagement member 24. Coupling member 22 bearing threaded portion 22a engageable with a pressure transducer is integrally provided in the interior of the lower end of dome 12.

At the time of priming, a priming liquid is taken into dome 12 through tube 26 and passage 20. Unlike the conventional type, the liquid pressure transducer dome embodying the present invention is characterized, in that when the priming liquid runs from tube 26 into dome 12 through passage 20, the priming liquid runs unidirectionally from passage 20 to the diaphragm, thus suppressing the occurrence of turbulent flows of the priming liquid and the consequent generation of air bubbles. The further merit of the present invention is that even if air bubbles arise in dome 12 because of the rapid flow of the priming liquid, air bubbles can be very easily drawn to the outside through porous diaphragm 16.

When priming is brought to an end, the pressure dome is connected to the pressure transducer. If, at this time, air happens to be retained between the pressure transducer and diaphragm 16, accurate pressure determinations are made impossible. Until now, therefore, it has been undertaken to ascertain by the naked eye whether air is left between the diaphragm and pressure transducer. If in such case the diaphragm is wholly rendered porous, the diaphragm will become opaque, thereby obstructing the recognition of retained air bubbles.

If, in the above-mentioned case, central region 16a which constitutes the greater part of diaphragm 16 is rendered transparent by thermal a ultrasonic fusion, and only circumferential region 16b is rendered gas-permeable, then it will be possible to attain not only the discharging of air but also to determine the presence of air bubbles. Particularly, because air bubbles tend to be retained in the junction between diaphragm 16 and dome 12, the above-mentioned process will be sufficient to attain the object of the present invention. However, if the type of transducer and flexibility of the diaphragm are properly selected, it may be possible to accurately measure the liquid pressure even when air is present between diaphragm 16 and the transducer. In such a case, it is not necessary to render diaphragm 16 transparent as above.

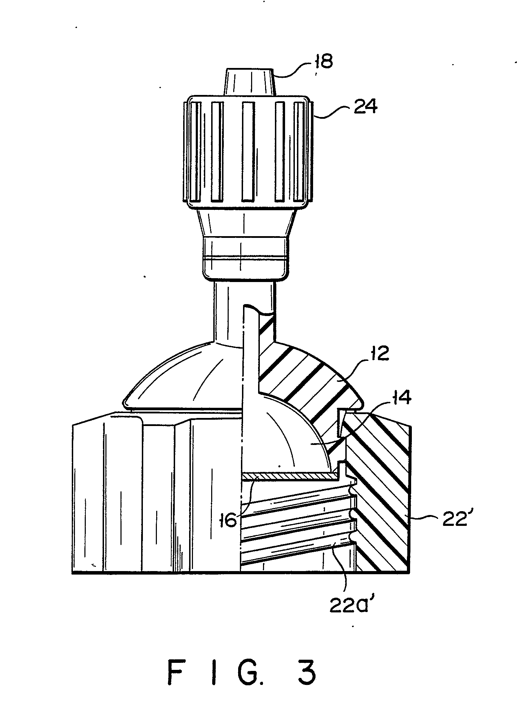

Description may now be made with reference to Fig. 3 of another modification of a liquid pressure transducer dome of the present invention. Referring to Fig. 3, coupling member 22' of the dome is formed of a cylindrical body where inner walls provided with threaded portion 22a, namely, the embodiment of Fig. 3 is of the so-called round lock type rotatably engaged with dome 12. The embodiment of Fig. 3 is characterized in that the transducer and dome 12 can be coupled together simply by the rotation of threaded coupling member 22'. Further advantage of the modification of Fig. 3 is that even if diaphragm 16 is displaced from the horizontal plane or screw 22a is not satisfactorily engaged, coupling between diaphragm 16 and transducer can be assured. Consequently it is possible to correctly determine static pressure and also to improve frequency properties observed in the determination of dynamic pressure. Threaded member 24 may also be of the round lock type.

The foregoing description refers to the particular embodiments of the present invention. However, the invention is not limited thereto; it is applicable to any other type of liquid pressure transducer dome. In other words, the present invention is applicable to any other type of liquid pressure transducer dome where liquid is made to flow, under pressure, against a diaphragm set at the bottom of a dome where the liquid is received, and the liquid pressure is converted to an electric signal by way of a liquid pressure transducer.

As mentioned above, the pressure transducer dome representing the present invention has a porous diaphragm which is pervious to gas but impervious to liquids. Thus, gases retained in the dome can be drawn to the outside through the diaphragm, therefore allowing for easy priming. Further, the dome of the invention dispenses with the need for a gas outlet port in the dome, and consequently ensures the simplification of the construction of a liquid pressure transducer dome.

## Claims

1. A liquid pressure transducer dome characterized by comprising:
a dome body provided with means for ensuring its connection to a liquid pressure transducer:
a diaphragm which comprises a film which is pervious to gas but impervious to liquids, and is set in the dome to transmit liquid pressure to the liquid pressure transducer; and
liquid passage means, provided in the dome, for conducting the liquid to the diaphragm,
said diaphragm discharging gases present in said liquid passage means therethrough, when a liquid is introduced into said dome body.

2. The dome according to claim 1, characterized in that the central portion of said diaphragm is rendered transparent or translucent.

3. The dome according to claim 1, characterized in that said diaphragm is prepared from a hydrophobic plastics material.

4. The dome according to claim 3, characterized in that said diaphragm is prepared from polyethylene, polypropylene, polytetrafluoroethylene, polyvinylidene fluoride or tetrafluoroethylene-hexafluoropropylene copolymer.

5. The dome according to claim 3, characterized in that said diameter of pores in the diaphragm ranges from 0.01 to 5 $\mu$m on the average.

F I G. 1

F I G. 2

F I G. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 252 126 (J.P. MANDL)<br>* Abstract; column 2, lines 38-41,48-55; column 3, lines 5-19; figures 1,3 * | 1,3,5 | A 61 B 5/02<br>G 01 L 7/08 |
| A | US-A-4 072 056 (ARNOLD ST. JACQUES LEE)<br>* Abstract; column 2, lines 1-28; column 2, line 53 - column 3, line 3; figures 1,2 * | 1-4 | |

TECHNICAL FIELDS SEARCHED (Int. Cl.4)

A 61 B
G 01 L

The present search report has been drawn up for all claims

| Place of search | Date of completion of the search | Examiner |
|---|---|---|
| THE HAGUE | 17-08-1987 | FERRIGNO, A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82